# EUROPEAN PATENT APPLICATION

(11) **EP 2 724 708 A1**
(43) Date of publication of application: **30.04.2014**
(21) Application number: 12189988.4
(22) Date of filing: 25.10.2012
(51) Int. Cl.: A61K 8/34, A61Q 5/04, A61Q 5/10, A61K 8/22

(54) **Aqueous oxidizing composition comprising an organic solvent**

(71) Applicant: Kao Germany GmbH, 64297 Darmstadt (DE)
(72) Inventor: Wood, Jonathan, 69469 Weinheim (DE); Weißbach, Kornelia, 65812 Bad Soden (DE)
(74) Representative: Grit, Mustafa

(57) **Abstract**

Present invention relates to an aqueous oxidizing composition comprising at least one oxidizing agent and at least one organic solvent. It has surprisingly been found out that an aqueous oxidizing composition comprising at least one oxidizing agent and at least one organic solvent does effectively recover disulfide bonds in hair after a reducing treatment and hair permanently shaped with the said composition has excellent curling or straightening efficiency and hair dyed with the use of the said composition is homogeneously dyed and appears to look more natural and feels natural upon touching in terms of softness, smoothness, and grip.

## Description

Present invention relates to an aqueous oxidizing composition comprising at least one oxidizing agent and at least one organic solvent.

Oxidizing compositions have been known in the hair cosmetic art and used in lightening hair color, hair coloring especially with oxidative dyestuff precursors and in permanent shaping hair involving a reducing step. In all these applications it has been observed that oxidative hair damage takes place. In order to achieve better results it is common to increase concentration of the oxidizing agent which even further increases the level of oxidative damage. On one hand, especially in permanent shaping hair reestablishing the broken disulfide bounds is vital in order to achieve acceptable and long lasting permanent shaping efficiency. On the other hand, in order to achieve beautiful colors it is vital to achieve required lightening level and in order to keep the oxidative damage to hair as low as possible, lightening and coloring has to be done in the shortest possible time.

With all previously available oxidizing agents in permanent shaping art, it has been observed that at relatively lower concentrations of oxidizing agent, the reestablishment efficiency of disulfide bonds is not always satisfactory and at relatively higher concentrations of oxidizing agent, although the recovery level of disulfide bonds is acceptable but hair color alteration is seen as a major disadvantage along with hair damage. There is obviously need for new oxidizing compositions which does not have one or more of the mentioned disadvantages and also has better and improved performance especially in oxidative coloring and permanent shaping processes.

In oxidative hair dyeing area, it has especially been observed that dyeing is required to be done in a shorter period of time and at oxidizing agent concentrations as low as possible in order to limit and/or reduce the oxidative damage to the hair. On the other hand, it is required to achieve homogeneous colors in the entire length of hair. It is known that level of damage is very much effecting the dyestuff penetration into hair and therefore the color achieved from an oxidative coloring composition. Despite the attempts to overcome color homogeneity on a hair comprising parts damaged at various levels, there is still need for additional technologies to overcome difficulties to achieve homogeneous coloration.

Inventors of the present invention has surprisingly found out that an aqueous oxidizing composition comprising at least one oxidizing agent and at least one organic solvent does effectively recover disulfide bonds in hair after a reducing treatment and hair permanently shaped with the said composition has excellent curling or straightening efficiency and hair dyed with the use of the said composition is homogeneously dyed and appears to look more natural and feels natural upon touching in terms of softness, smoothness, and touch.

Accordingly, the first object of the present invention is an aqueous composition comprising at least one oxidizing agent and at least one organic solvent.

Second object of the present invention is the use of an aqueous composition comprising at least one oxidizing agent and at least one organic solvent for permanent shaping hair. In this respect further object of the present invention is the use of an aqueous composition comprising at least one oxidizing agent and at least one organic solvent for reducing sulfhydryl (SH) groups in hair at the end of a permanent shaping process.

Third object of the present invention is the use of an aqueous composition comprising at least one oxidizing agent and at least one organic solvent for coloring hair.

The fourth object of the present invention is a process for permanent shaping hair comprising the steps of
a- treating hair with an aqueous composition comprising at least one reducing agent preferably at a concentration higher than 2% by weight calculated to the total of the composition,
b- treating hair with an aqueous composition at least one oxidizing agent and at least one organic solvent.

In the above process, curlers are put on the hair, in case of a curling process - hair is mechanically stressed - before treating hair with aqueous reducing composition, or during application or after application of aqueous reducing composition. The curlers are then taken off from hair before or during or after application of oxidizing composition or at the end of the processing time with oxidizing composition of the present invention.

The fifth object of the present invention is a hair two part hair coloring composition, A and B, which are mixed prior to application onto hair wherein part A is an aqueous composition and comprising at least one hair dye and Part B is an aqueous composition comprising at least one oxidizing agent and at least one organic solvent.

Further object of the present invention is a kit for hair comprising two or more compositions wherein one of the compositions is an aqueous composition comprising at least one oxidizing agent and at least one organic solvent.

Aqueous oxidizing composition of the present invention comprises at least one oxidizing agent at a concentration of 0.5 to 20%, preferably 1 to 15%, more preferably 1 to 12%, most preferably 2 to 9% by weight calculated to the total composition.

Suitable oxidizing agents are hydrogen peroxide, urea peroxide, melamin peroxide and sodium bromate. Most preferred is hydrogen peroxide. The pH of the composition comprising hydrogen peroxide is in the range of 2 to 5, preferably 2.5 to 4 and more preferably 3 to 4 whereas the pH of the composition comprising sodium bromate is in the range of 6.5 to 7.5.

Aqueous composition comprises at least one organic solvent, preferably at least one hair swelling organic solvent. Examples of such compounds may include lower alkylene carbonates, aromatic alcohols and N-alkylpyrrolidones. More specifically, examples of the lower alkylene carbonates include ethylene carbonate and propylene carbonate. Examples of the aromatic alcohols include benzyl alcohol, benzyloxyethanol, β-phenylethyl alcohol, cinnamyl alcohol, phenyl propanol, α-methylbenzyl alcohol, dimethyl benzyl carbinol, phenoxyethanol and p-anisyl alcohol. Examples of the N-alkylpyrrolidones include N-methylpyrrolidone, N-ethylpyrrolidone and N-octylpyrrolidone. The composition can comprise at least one organic solvent as well as mixture of organic solvents.

Preferably the composition comprises at least one aromatic organic solvent and most preferably it is selected from benzyl alcohol and phenoxyethanol and their mixtures.

Total concentration of organic solvent in the composition is in the range of 0.05 to 20%, preferably 0.1 to 15%, more preferably 0.25 to 10%, most preferably 0.5 to 7.5% and in particular 1 to 5% by weight calculated to the total of the composition.

The oxidizing composition of the present invention preferably comprises an organopolysiloxane wherein at least one silicon atom is linked to an alkylene group having a hetero-atom, in particular a nitrogen atom, with a poly-(N-acyl alkyleneimine) units of the formula 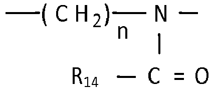 wherein n is a number from 1 to 5 and R₁₄ is hydrogen, a C₁-C₁₂-alkyl or cycloalkyl, aralkyl or aryl group.

Preferred organopolysiloxane polymers are those of the type disclosed in EP-A 640 643, in particular optionally quaternized aminoalkyl, in particular aminopropyl dimethyl polysiloxane/polyethyl oxazoline copolymers of the formula 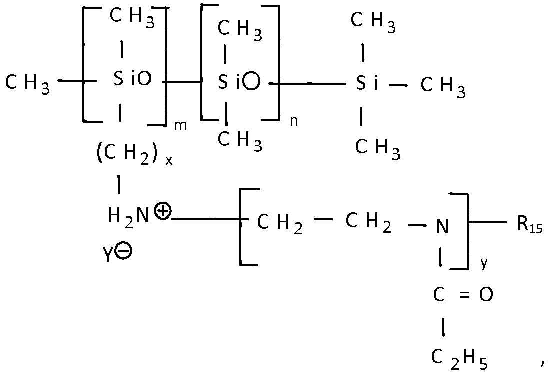 wherein m and n each are numbers from 20 to 10,000, in particular 50 to 7,000, especially 100 to 5,000, x is a number between 1 and 5, preferably 3, and y is a number from 5 to 30, R₁₅ is a C₁-C₁₂-alkyl or aryl group, in particular a methyl, ethyl or benzyl group, and Y⁻ is an anion.

Especially suited are the organopolysiloxanes disclosed under the terms A-1, A-2 and A-3 on pages 12 to 13 of EP-A 640 643. The proportion of graft copolymers in the hair colouring compositions according to the invention ranges from 0.05 % to 5 %, preferably 0.1 % to 2.5 %, in particular 0.5 % to 1.5 % by weight, calculated to the total composition.

Another compound that may be comprised in the aqueous oxidizing composition of the present invention is a ceramide type of compounds according to the general formula 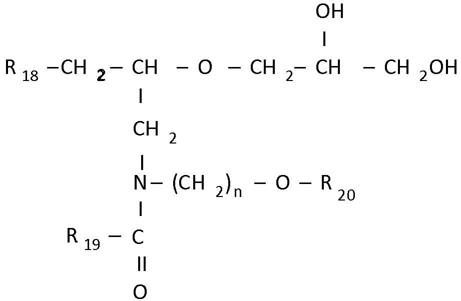 where R₁₈ and R₁₉ are independent from each other alkyl- or alkenyl group with 10 to 22 carbon atoms, R₂₀ is methyl, ethyl, n-propyl or isopropyl group and n is a number between 1 to 6, preferably 2 or 3. The most preferred compounds is cetyl PG hydroxyethyl palmitamide from Kao Corporation. The concentration of the ceramide type of compound in compositions of the present invention can be in the range of 0.01 to 2 and especially 0.01 to 1% by weight calculated to the total composition.

The aqueous compositions of the present invention further preferably comprise one or more ubiquinone of the formula. 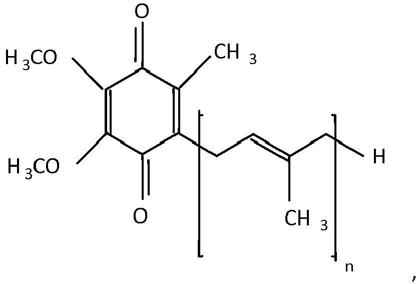 wherein n is a number from 1 to 10. The concentration of ubichinones in the compositions of the present invention can vary between 0.001% and 10% by weight, calculated to the total composition.

The composition of the present invention may be in the form of solutions, gels and emulsions. It is noted that in permanent shaping processes and especially curling hair and when oxidizing composition is applied when the curlers are on the hair, relatively low viscosity compositions are required for the best performance. In other cases the composition should have the consistency which makes the application and processing suitably possible and therefore may have various consistencies.

In case the composition is in the form of gel, one or more gelling agent is suitable be comprised in the compositions. Suitable ones are any known in the art which are water soluble for example cellulose derivatives such as hydroxyethyl cellulose, hydroxyethyl methyl cellulose and their cationic derivatives such as Polyquaterniums 10 and 67, guar gums such as hydroxyethyl guar gum, guar hydroxypropyl trimonium chloride, xanthan gum, acrylates such as carbomer and alkyl acrylates. Concentration of one or more polymers should be adjusted in accordance with the required viscosity. Typically the concentration of one or more polymers may be in the range of 0.01 % to 5 %, preferably 0.05 % to 2.5 %, more preferably 0.1 % to 2 %, most preferably 0.1 % to 1.5 % and in particular 0.2 % to 1 % by weight, calculated to the total composition.

The composition of the present invention can advantageously comprise one or more surfactant selected from anionic, non-ionic, cationic and amphoteric surfactants. Preferred surfactants are anionic, non-ionic and cationic ones and especially preferred are the mixture of anionic and non-ionic surfactants and mixture of cationic non-ionic surfactants at any ratio.

In principal any anionic surfactant is suitable within the meaning of the present invention. Nonlimiting examples are anionic surfactants of the sulfate, sulfonate, carboxylate and alkyl phosphate type, especially, of course, those customarily used as emulsifiers, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates and their salts.

Further suitable surfactants are alkyl polyether carboxylic acids and the salts thereof of the formula

R₅-(C₂H₄O)ₙ-O-CH₂COOX,

wherein R₅ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 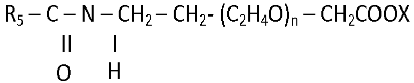 wherein R₅ and X have the above meanings, and n is in particular a number from 1 to 10, preferably 2.5 to 5.

Among the anionic surfactants most preferred are alkyl sulfates and/or alkyl ether sulfates and among them sodium lauryl or laureth sulfates and their mixtures are most preferred.

Suitable non-ionic surfactants are alkyl polyglucosides of the general formula

R₆-O-(R₇O)ₙO-Zₓ

wherein R₆ is an alkyl group with 8 to 18 carbon atoms, R₇ is an ethylene or propylene group, Z is a saccharide group with 5 to 6 carbon atoms, n is a number from 0 to 10 and x is a number between 1 and 5. Examples are decyl polyglucoside and cocoyl polyglucoside, both beeing commercially available.
Further nonionic surfactant components are, for example, long-chain fatty acid mono- and dialkanolamides, such as coco fatty acid monoethanolamide and myristic fatty acid monoethanolamide.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}".

Further nonionic surfactants in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

Among the non-ionic surfactants mentioned above fatty alcohol ethoxylates and fatty acid alkanolamides and their mixtures at any weight ratio are the most preferred ones.

As a rule any mono alkyl quaternary ammonium surfactants is suitable for the compositions of the present invention as cationic surfactant. With the term mono alkyl it is meant that quaternary ammonium surfactant includes only one alkyl chain which has more than 8 C atoms. The term does not exclude that the quaternary ammonium surfactant includes further short alkyl chains, C₁ to C₄, present in the molecule.

Preferably at least one mono alkyl quaternary ammonium surfactant is selected from the compounds with the general formula 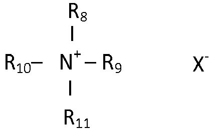 where R₈ is saturated or unsaturated, branched or non-branched alkyl chain with 8-22 C atoms or

R₁₂CONH(CH₂)ₙ

where R₁₂ is saturated or unsaturated, branched or non-branched alkyl chain with 7-21 C atoms and n has typical value of 0 - 4 or

R₁₂COO(CH₂)ₙ

where R₁₂ is saturated or unsaturated, branched or non-branched alkyl chain with 7-21 C atoms and n has typical value of 0 - 4, and

R₉, R₁₀ and R₁₁ are independent from each other lower alkyl chain with 1 to 4 carbon atoms, hydroxyl alky chain with 1 to 4 C atoms, or ethoxy or propoxy group with number of ethoxy or propoxy groups varying in the range of 1 to 4, and X is chloride, bromide or methosulfate.

Suitable cationic surfactants and or conditioning agents are, for example, long-chain quaternary ammonium compounds which can be used alone or in admixture with one another, such as cetyl trimethyl ammonium chloride, myristoyl trimethyl ammonium chloride, behentrimonium chloride, trimethyl cetyl ammonium bromide, stearyl trimethyl ammonium chloride, stear trimonium chloride, stearamidopropyltrimethylammonium chloride, stearamidopropyl trimonuim chloride.

Surfactants are included into the composition at a total concentration of 0.05 to 10%, preferably 0.1 to 7.5% and more preferably 0.2 - 5%, and most preferably 5 to 4% by weight, calculated to the total composition.

In case the composition of the present invention is an emulsion it preferably comprises one or more fatty alcohol of the general formula

R₄-OH

wherein R₄ is a linear or branched, saturated or unsaturated alkyl chain with 12 to 22 C atoms and at least one emulsifier selected from anionic, non-ionic, cationic and amphoteric surfactants.

Suitable fatty alcohols are myristyl alcohol, cetyl alcohol, stearyl alcohol and behenyl alcohol and their mixtures. Most preferred is the mixture of cetyl and stearyl alcohol also known as cetearyl alcohol.

The concentration of one or more fatty alcohols is in the range of 0.25 to 10%, preferably 0.5 to 7.5%, more preferably 1 to 7.5% and most preferably 1 to 5% by weight calculated to total composition.

Compositions of the present invention may further comprise other compounds found in customarily in such compositions. They may suitably be chelating agents, preservatives, acids in order to adjust the pH of the compositions, peroxide preserving agents such as acetaminophen and salicylic acid.

In case the composition of the preset invention is used in a permanent shaping process, hair is treated first with an aqueous composition comprising at least one reducing compound preferably at a concentration 0.5 to 15 %, preferably 1.0 to 12.5%, more preferably 2.0 to 12.5%, most preferably 2.5 to 12.5% by weight, calculated to total composition. Preferred are thioglycolic acid and thiolactic acid as well as the salts thereof, in particular the ammonium and ethanolamine salts. Further useful thio compounds are in particular cysteine or the hydrochloride thereof, homocysteine, cysteamine, N-acetyl cysteine, thioglycerol, ethanediol monothioglycolate, 1.2-propyleneglycol monothioglycolate (see also WO-A 93/1791), 1-3-propanediol monothioglycolate or the isomer mixture resulting therefrom, 1.3-butanediol and 1.4-butanediol monothioglycolate and the isomer mixtures therefrom, polyethylene glycol, such as di-, tri- and tetraethyleneglycol monothioglycolates, glycerol monothiolactate and further thio acids and the esters thereof, as well as mixtures thereof. The use of inorganic reducing sulfur compounds such as sodium hydrogen sulfite is basically also possible.

In case the composition of the present invention is used in hair dyeing process, the composition is mixed with a composition comprising at least one hair dye (composition A). Suitable hair dyes are oxidative dye precursors and direct dyes of cationic, anionic and nonionic nitro dyes.

Suitable non-limiting oxidative dyestuffs precursors are tetraaminopyrimidines, in particular 2,4,5,6-tetraaminopyrimidine and the lower alkyl derivatives thereof; suitable triaminohydroxypyrimidines are, for example 4-hydroxy-2,5,6-triaminopyrimidine, 2-hydroxy-4,5,6-triaminopyrimidine and 5-hydroxy-2,4,6-triaminopyrimidine; suitable mono- and diamino dihydroxypyrimidines are, for example, 2,6-dihydroxy-4,5-diaminopyrimidine, 2,4-diamino-6-hydroxy-pyrimidine or 4,6-dihydroxy-2,5-diaminopyrimidine or the water-soluble salts thereof, aminophenol derivatives such as 4-aminophenol, 4-amino-3-methylphenol, 2-chloro-4-aminophenol, 2,6-dichloro-4-aminophenol, 2,4-diamino-phenol, 2,6-dibromo-4-aminophenol and/or 2-aminophenol and water-soluble salts thereof, furthermore, phenylenedimanine derivatives such as 2,5-diamino-toluene, 2-n-propyl or 2-ethyl-p-phenylene-diamine, 2,6-dimethyl-p-phenylenediamine, 2-(2,5-diaminophenyl) ethanol, 1-amino -4-bis-(2'-hydroxyethyl)aminobenzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene or the water-soluble salts thereof, pyrazole derivatives such as 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 1-methyl-4,5-diaminopyrazole, 1-methylethyl-4,5-diaminopyrazole, 1-phenylmethyl-4,5-diaminopyrazole, 1-methyl-4,5-diaminopyrazole, 1-(4-methylphenyl)methyl-4,5-diaminopyrazole, 1-methyl-3-phenyl-4,5-diaminopyrazole and the water-soluble salts. The use of the above mentioned oxidative dye precursors as mixture is also customary in hair coloring area.

The total concentration of the oxidation dyestuff precursors and/or their water soluble salts customarily ranges between about 0.05 % and 5 %, preferably 0.1 % and 4 %, in particular 0.1 % to 3% by weight, calculated to the total hair dyeing composition prior to mixing with oxidizing agent of the present invention.

The composition A can also comprises optionally at least one coupling substance, which can be selected from resorcinol, 2-methyl resorcinol, 4-chlororesorcinol, 2-amino-4-chlorophenol, 5-amino-4-methoxy-2-methylphenol, 3-amino-phenol, 1-methyl-2-hydroxy-4-aminobenzene, 3-N,N-dimethyl aminophenol, 2.6-dihydroxy-3.5-dimethoxypyridine, 5-amino-3-methylphenol, 6-amino-3-methylphenol, 3-amino-2-methylamino-6-methoxypyridine, 2-amino-3-hydroxy-pyridine, 2-dimethyl-amino-5-aminopyridine, 2,6-diaminopyridine, 1,3-diamino- benzene, 1-amino-3-(2'-hydroxyethylamino)benzene, 1-amino-3-[bis(2'-hydroxy-ethyl) amino]benzene, α-naphthol, 4,6-dichlororesorcinol, 1,3-diamino-toluene, 1-hydroxy naphthalene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1.2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof.

However, this shall not exclude the addition of further developing and coupling substances. In the preferred embodiment of the present invention composition comprise additionally at least one coupling agent.

The weight proportion of the named developing substances to the coupling substances ranges between about 1 : 8 to 8 : 1, preferably about 1 : 5 to 5 : 1, in particular 1 : 2 to 2 : 1. In the hair dyeing compositions according to the invention, the coupling substance(s) as reaction partners of the developing substance(s) are present in approximately the same molecular proportions as the developing substances, i.e. in amounts from 0.01 % to 5.0 %, preferably 0.05 % to 4 %, in particular 0.1 % to 3 % by weight, calculated to the total composition prior to mixing with oxidizing agent, whereby these figures are always related to the proportion of free base.

The composition A can comprise additionally direct dyes of neutral, cationic and anionic character. Some examples to suitable cationic dyes are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14 and Basic Yellow 57. According to the invention, suitable cationic dyestuffs are in principal those any available on the market for cosmetic hair colouring applications. For this purpose, special reference is made to the PCT application WO 95/15144 of Ciba-Geigy AG. The content of the PCT application WO 95/15144 is by reference incorporated here.

Examples to suitable direct acting anionic dyes are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Some examples to those suitable neutral dyes (HC dyes), so called nitro dyes, are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder, etc.

According to the invention, the composition A comprises direct hair dyes at a total concentration of 0.01 to 5%, preferably 0.05 to 3%, more preferably 0.1 to 2% by weight calculated to total composition excluding the oxidative composition.

It should be noted that the dyeing composition may be in any form known in the art, such as solutions, gels and emulsions and may comprise accordingly any compounds found customarily in such compositions.

Following examples are to illustrate the invention, but not to limit it.

### Example 1

| | % by weight |
|---|---|
| Hydrogen peroxide | 2.0 |
| Phosphoric acid | q.s. to pH 2.5 |
| Phenoxyethanol | 3.0 |
| Salicylic acid | 0.2 |
| Water | q.s. to 100 |

The above composition was used in a permanent hair shaping process. For this purpose a 20 cm long hair streak (weighting approximately 10 g) was first shampooed and towel dried and a composition comprising 8% by weight of ammonium thioglycolate was applied to the hair and after processing 20 min at ambient temperature, rinsed off from hair and hair was towel dried. The hair was subsequently treated with the above composition again at ambient temperature for 10 min and rinsed off from hair and hair was dried with a hair drier after towel drying.

For comparative purposes the above process was repeated with a second hair streak wherein in the oxidizing step the above composition without phenoxyethanol was used. Phenoxyethanol was replaced with water.

Remaining free SH groups were analyzed for both streaks and it was observed that the steak treated with inventive composition had much less SH groups than that of the comparative streak.

The free SH group content of the hair was analyzed as described on page 104 in the dissertation of Dr. R. Sauer published in September 2001.

Similar results were observed with the following compositions.

### Example 2

| | % by weight |
|---|---|
| Hydrogen peroxide | 2.0 |
| Phosphoric acid | q.s. to pH 2.5 |
| Coenzyme Q10 | 0.1 |
| Polysilicone-9 | 0.5 |
| Benzyl alcohol | 2.0 |
| Salicylic acid | 0.2 |
| Water | q.s. to 100 |

### Example 3

| | % by weight |
|---|---|
| Hydrogen peroxide | 2.0 |
| Phosphoric acid | q.s. to pH 2.5 |
| Coenzyme Q10 | 0.1 |
| Polysilicone-9 | 0.5 |
| Cetyl-PG hydroxyethyl palmitamide | 0.1 |
| Benzyl alcohol | 1.0 |
| Salicylic acid | 0.2 |
| Water | q.s. to 100 |

### Example 4

| | % by weight |
|---|---|
| Hydrogen peroxide | 2.0 |
| Phosphoric acid | q.s. to pH 2.5 |
| Coenzyme Q10 | 0.1 |
| Polysilicone-9 | 0.5 |
| Phenoxyethanol | 1.5 |
| Acetaminophen | 0.2 |
| Water | q.s. to 100 |

### Example 5

| | % by weight |
|---|---|
| Hydrogen peroxide | 2.0 |
| Phosphoric acid | q.s. to pH 2.5 |
| Coenzyme Q10 | 0.1 |
| Polysilicone-9 | 0.5 |
| Cetyl-PG hydroxyethyl palmitamide | 0.1 |
| Phenoxyethanol | 1.0 |
| Salicylic acid | 0.2 |
| Water | q.s. to 100 |

### Example 6

Example 1 was used for coloring hair with a composition comprising the following dyestuffs.

| | % by weight |
|---|---|
| p-toluenediamine | 1.0 |
| Resorcinol | 0.6 |

The pH of the composition was 9.5 after mixing with oxidizing composition of example 1 at a weight ratio of 1:1. It was observed that hair was homogeneously dyed with the oxidizing composition of the present invention. The comparative streak dyed with the oxidizing composition without phenoxyethanol was less homogeneous.

Similar results were obtained with the following compositions.

### Example 7

| | % by weight |
|---|---|
| 1-Hydroxyethyl-4,5-diaminopyrazole | 0.63 |
| 4-Amino-2-hydroxytoluene | 0.33 |

Homogeneous orange colour was observed.

### Example 8

| | % by weight |
|---|---|
| Toluene-2,5-diamine | 0.45 |
| Resorcinol | 0.20 |
| 1,3-Bis-(2,4-diaminophenoxy)propane | 0.10 |
| HC Yellow 2 | 0.005 |

Homogeneous blue-brown colour was observed.

### Example 8

| | % by weight |
|---|---|
| Toluene-2,5-diamine | 0.50 |
| 4-Amino-2-hydroxytoluene | 0.50 |
| 1-Naphthol | 0.50 |
| p-Aminophenol | 0.50 |

Homogeneous reddish-copper colour was observed.

## Claims

1. Aqueous composition for hair **characterized in that** it comprises at least one oxidizing agent ad at least one organic solvent.

2. The composition according to claim 1 **characterized in that** the oxidizing agent is hydrogen peroxide.

3. The composition according to claims 1 and 2 **characterized in that** oxidizing agent is present at a concentration 0.5 to 20% by weight calculated to the total composition.

4. The composition according to any of the preceding claims **characterized in that** at least one organic solvent is a hair swelling organic solvent.

5. The composition according to any of the preceding claims **characterized in that** at least one organic solvent is an aromatic organic solvent.

6. The composition according to any of the preceding claims **characterized in that** at least one organic solvent is benzyl alcohol and/or phenoxyethanol.

7. The composition according to any of the preceding claims **characterized in that** at least one organic solvent is present at a concentration of 0.05 to 20%, preferably 0.1 to 15%, more preferably 0.25 to 10%, most preferably 0.5 to 7.5% and in particular 1 to 5% by weight calculated to the total of the composition.

8. The composition according to any of the preceding claims **characterized in that** it comprises one or more of the following ingredients
a- an organopolysiloxane wherein at least one silicon atom is linked to an alkylene group having a hetero-atom, in particular a nitrogen atom, with a poly-(N-acyl alkyleneimine) units of the formula 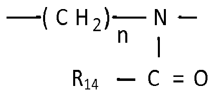 wherein n is a number from 1 to 5 and R₁₄ is hydrogen, a C₁-C₁₂-alkyl or cycloalkyl, aralkyl or aryl group, and/or
b- a ceramide type of compounds according to the general formula 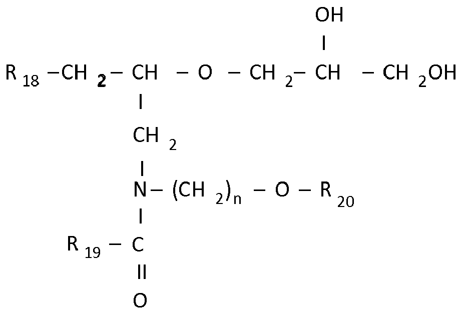 where R₁₈ and R₁₉ are independent from each other alkyl- or alkenyl group with 10 to 22 carbon atoms, R₂₀ is methyl, ethyl, n-propyl or isopropyl group and n is a number between 1 to 6, preferably 2 or 3, and/or
c- an ubiquinone of the formula. 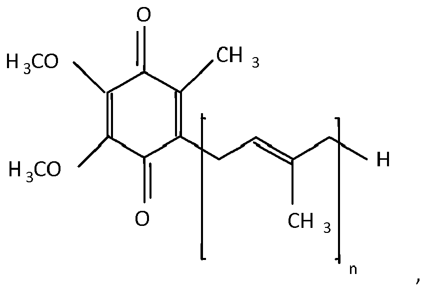 wherein n is a number from 1 to 10.

9. The composition according to any of the preceding claims **characterized in that** it comprises at least one surfactant, preferably selected from anionic, non-ionic and cationic ones.

10. The composition according to any of the preceding claims **characterized in that** it comprises at least one fatty alcohol.

11. Use of the aqueous composition according to claims 1 to 10 for permanent shaping hair, especially for reducing sulfhydryl (SH) groups in hair at the end of a permanent shaping process

12. Use of the composition according to claims 1 to 10 for coloring hair.

13. Process for permanent shaping hair **characterized in that** it comprising the steps of
a- treating hair with an aqueous composition comprising at least one reducing agent preferably at a concentration higher than 2% by weight calculated to the total of the composition,
b- treating hair with the composition according to claims 1 to 10.

14. Two part hair coloring composition, A and B, which are mixed prior to application onto hair wherein part A is an aqueous composition and comprising at least one hair dye and Part B is an aqueous composition according to claims 1 to 10.

15. Kit for hair comprising two or more composition **characterized in that** one of the compositions is a composition according to claims 1 to 10.
